(19) Europäisches Patentamt
European Patent Office
Office européen des brevets

(11) **EP 4 597 083 A1**

(12) **EUROPEAN PATENT APPLICATION**
published in accordance with Art. 153(4) EPC

(43) Date of publication:
**06.08.2025 Bulletin 2025/32**

(21) Application number: **23879436.6**

(22) Date of filing: **07.08.2023**

(51) International Patent Classification (IPC):
**G01N 21/88** (2006.01)    **G01N 21/89** (2006.01)

(52) Cooperative Patent Classification (CPC):
**G01N 21/88; G01N 21/89**

(86) International application number:
**PCT/JP2023/028775**

(87) International publication number:
**WO 2024/084783 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.10.2022 JP 2022166268**

(71) Applicant: **Hamamatsu Photonics K.K.
Hamamatsu-shi, Shizuoka 435-8558 (JP)**

(72) Inventors:
• **MATSUDA Shunsuke
Hamamatsu-shi, Shizuoka 435-8558 (JP)**
• **KONDO Hideyuki
Hamamatsu-shi, Shizuoka 435-8558 (JP)**
• **TSUCHIYA Kunihiko
Hamamatsu-shi, Shizuoka 435-8558 (JP)**

(74) Representative: **Hoffmann Eitle
Patent- und Rechtsanwälte PartmbB
Arabellastraße 30
81925 München (DE)**

(54) **IMAGE ACQUISITION DEVICE, DETECTION DEVICE, AND IMAGE ACQUISITION METHOD**

(57)    An image acquisition device 1 includes an illumination device 2 that irradiates a target object S with polarized light, an imaging device 7 that detects light that is specularly reflected by the target object S in the polarized light, and acquires a plurality of pieces of image data including different polarization components of the light, and an image processing device 8 that detects a foreign substance that is present on the target object S and through which the polarized light is transmitted, based on the plurality of pieces of image data.

*Fig.1*

EP 4 597 083 A1

## Description

### Technical Field

One aspect of an embodiment relates to an image acquisition device, an inspection device, and an image acquisition method.

### Background Art

In the related art, a device has been known, which irradiates an inspection target object with light and detects light transmitted through the inspection target object to inspect the inspection target object. For example, in a device disclosed in Patent Literature 1, a distribution of birefringence phase differences of an inspection target object is calculated from an intensity signal detected by an image sensor, and a quality of the inspection target object is determined based on the distribution. With the device having such a configuration, it is possible to inspect the inspection target object having birefringence.

### Citation List

### Patent Literature

[Patent Literature 1] Japanese Unexamined Patent Publication No. 2020-190514

### Summary of Invention

### Technical Problem

In the related-art device described above, since light transmitted through the inspection target object is detected, it tends to be difficult to detect an object having a property of reflecting the light. Therefore, it is required to detect an object having a property of reflecting the light on the inspection target object with high accuracy.
Therefore, one aspect of the embodiment is made in view of such an issue, and an object of the aspect is to provide an image acquisition device, an inspection device, and an image acquisition method, with which an object present on a target object and having a property of reflecting the light can be detected with high accuracy.

### Solution to Problem

A first aspect of the embodiment relates to an image acquisition device including: a light irradiation device that irradiates a target object with polarized light; an imaging device that detects light that is specularly reflected by the target object in the polarized light, and acquires image data including different polarization components of the light; and an image processing unit that detects an object that is present on the target object and through which the polarized light is transmitted, based on a plurality of images.
Alternatively, a second aspect of the embodiment relates to an image acquisition method including: a light irradiation step of irradiating a target object with polarized light; an imaging step of detecting light that is specularly reflected by the target object in the polarized light, and acquiring image data including different polarization components of the light; and an image processing step of detecting an object that is present on the target object and through which the polarized light is transmitted, based on a plurality of images.
According to the first aspect or the second aspect, it is possible to evaluate a distribution of polarization states of the light that is specularly reflected by the target object in the polarized light, based on the detected image data. As a result, it is possible to detect the object present on the target object and having properties of transmitting and reflecting the polarized light with high accuracy.
Alternatively, a third aspect of the embodiment relates to an inspection device including: the image acquisition device according to any one of the first aspect; a transport device that transports the target object in a predetermined direction; and an inspection processing unit that inspects the target object based on data output from the image acquisition device.
According to the third aspect, it is possible to efficiently detect the objects present on a plurality of target objects and having the properties of transmitting and reflecting the polarized light while the plurality of target objects are being transported.

## Advantageous Effects of Invention

According to any aspect of the present invention, it is possible to detect the object present on the target object and having the property of reflecting the light with high accuracy.

## Brief Description of Drawings

Fig. 1 is a schematic configuration diagram illustrating an image acquisition device 1 according to an embodiment.
Fig. 2 is a diagram illustrating an image of reflected light generated in a target object S in a case in which the image acquisition device 1 of Fig. 1 is used.
Fig. 3 is a diagram illustrating a plurality of pieces of image data acquired for the same target object S by an image processing device 8.
Fig. 4 is a graph illustrating a change in brightness in one pixel with respect to a rotation angle of a rotating waveplate 4 in the image data illustrated in Fig. 3.
Fig. 5 is a schematic configuration diagram illustrating an inspection system 100 according to the embodiment.
Fig. 6 is a flowchart illustrating a procedure of an inspection method for the target object S using the inspection system 100.
Fig. 7 is a diagram illustrating an original image of the reflected light acquired by a camera 6 of the inspection system 100.
Fig. 8 is a diagram illustrating an inspection result image that is a specular reflection distribution acquired by a computer 12 of the inspection system 100.
Fig. 9 is a diagram illustrating an inspection result image that is a linear polarization distribution acquired by the computer 12 of the inspection system 100.
Fig. 10 is a graph illustrating a brightness distribution at a position of a line P2 on three images illustrated in Figs. 7 to 9.
Fig. 11 is a schematic configuration diagram illustrating an image acquisition device 1A according to a first modification example.
Fig. 12 is a diagram illustrating an original image of the reflected light acquired by the inspection system 100.
Fig. 13 is a diagram illustrating an inspection result image that is a circular polarization distribution acquired by the inspection system 100.
Fig. 14 is a graph illustrating a brightness distribution at a position of a line P3 on two images illustrated in Figs. 12 and 13.
Fig. 15 is a schematic configuration diagram illustrating an image acquisition device 1B according to a second modification example.
Fig. 16 is a diagram illustrating an original image of the reflected light acquired by the inspection system 100.
Fig. 17 is a diagram illustrating an inspection result image acquired by the inspection system 100.
Fig. 18 is a graph illustrating a brightness distribution at a position of a line P4 on two images illustrated in Figs. 16 and 17.
Fig. 19 is a diagram illustrating a configuration of an imaging device 201 according to a modification example.

## Description of Embodiments

Hereinafter, embodiments of the present invention will be described in detail with reference to the accompanying drawings. In the description, the same elements or the elements having the same functions are denoted by the same reference numerals, and the duplicate description will be omitted.

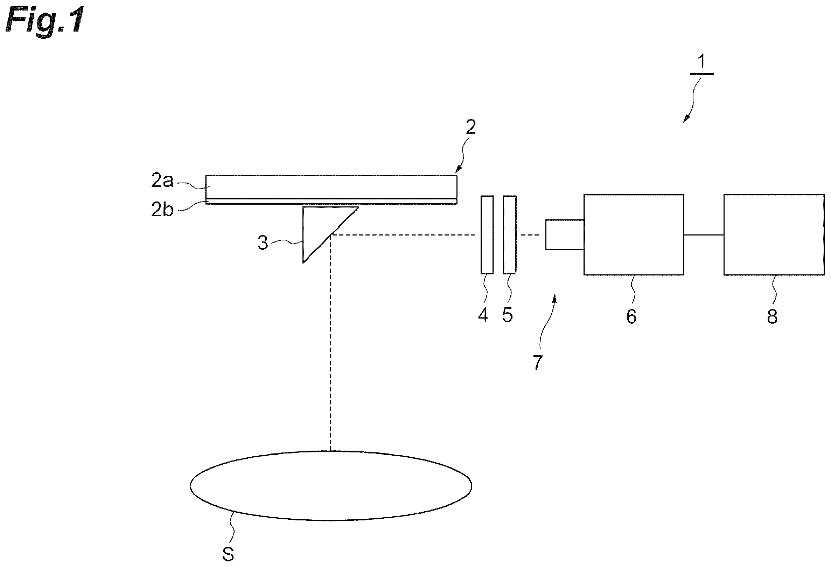

Fig. 1 is a schematic configuration diagram illustrating an image acquisition device 1 according to the embodiment. The image acquisition device 1 is a device that acquires image data of a target object for the purpose of inspecting whether or not a foreign substance is present on the target object such as food. However, the target object, which is a target of an inspection performed by the image acquisition device 1, may be other articles such as electronic components, in addition to food represented by beef, pork, chicken, lamb, and processed food. In Fig. 1, a light propagation path is indicated by a dotted line, and a transmission path of data such as image data is indicated by a solid line.

The image acquisition device 1 includes an illumination device (light irradiation device) 2, a right angle prism mirror 3, an imaging device 7 including a rotating waveplate 4, a polarizing plate 5, and a camera 6, and an image processing device (image processing unit) 8. Hereinafter, each component of the image acquisition device 1 will be described in detail.

The illumination device 2 is configured by a body portion 2a that performs irradiation by diffusing unpolarized light and a polarizing sheet 2b attached in proximity to a light irradiation surface of the body portion 2a, and performs irradiation by diffusing light in a predetermined polarization state (light polarized in a predetermined polarization state) toward a target object S. Examples of a light emitting device incorporated in the body portion 2a include an LED, a superluminescent diode (SLD), a laser, and a halogen lamp. A shape of the light irradiation surface of the body portion 2a may be a planar shape or may be a curved surface shape such as a spherical surface. In the present embodiment, the illumination device 2 performs the irradiation with circularly polarized light (light polarized to be circularly polarized light).

The right angle prism mirror 3 is provided adjacent to the light irradiation surface side of the illumination device 2. The right angle prism mirror 3 is an optical element having a function of reflecting light, which is generated by the polarized light from the illumination device 2 being reflected by the target object S, toward the camera 6. A mirror disposed at an angle of 45 degrees may be used instead of the right angle prism mirror 3.

The camera 6 provided in the imaging device 7 is an imaging element that is disposed at a position at which the light from the target object S reflected by the right angle prism mirror 3 can be detected via the rotating waveplate 4 and the polarizing plate 5 and detects a two-dimensional image of the light reflected by the target object S to acquire image data. As the camera 6, a complementary metal-oxide-semiconductor (CMOS) camera, a charge-coupled device (CCD) camera, or the like is used. In a case in which the target object S is transported in a predetermined direction by a transport device, a line sensor camera or a time delay integration (TDI) sensor camera may be used as the camera 6. In addition, the rotating waveplate 4, the polarizing plate 5, and the camera 6 may be configured by a polarization camera that has polarizer plates different for each pixel and can acquire an image including a plurality of polarization components.

The rotating waveplate 4 provided in the imaging device 7 is an optical element that is disposed between the right angle prism mirror 3 and the camera 6 and delays a phase of a one-direction polarization component of the light reflected by the right angle prism mirror 3. The rotating waveplate 4 is disposed, for example, at an angle at which the phase of the one-direction polarization component of the light is delayed by 90 degrees, but the phase value to be delayed is not limited to this. The rotating waveplate 4 is supported to be rotatable with a direction along an incidence direction of the light from the right angle prism mirror 3 as a rotation axis such that a polarization direction in which the phase is delayed is changed.

The polarizing plate 5 provided in the imaging device 7 is disposed between the rotating waveplate 4 and the camera 6. The polarizing plate 5 is an optical element that allows passage of a component of the linearly polarized light in a fixed direction in the light that is reflected from the right angle prism mirror 3 and then passes through the rotating waveplate 4.

The image processing device 8 is a device that detects the foreign substance on the target object S by receiving the image data acquired by the camera 6. Physically, the image processing device 8 is a computing device (computer or the like) that incorporates a central processing unit (CPU) or a graphics processing unit (GPU) as a processor, a random-access memory (RAM) or a read-only memory (ROM) as a recording medium, a communication module, an input/output module, and the like. In addition, the image processing device 8 may be configured by a field programmable gate array (FPGA) or an application-specific integrated circuit (ASIC). The image processing device 8 may acquire the image data from the camera 6 via a cable, or may acquire the image data from the camera 6 by wireless communication. A detection function of the image processing device 8 will be described later.

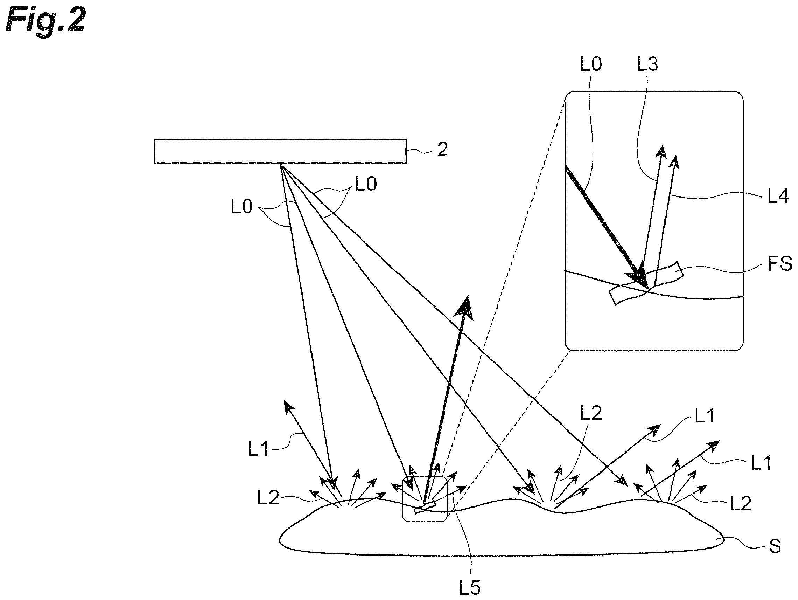

Here, a mechanism for detecting reflected light from the target object S in the image acquisition device 1 will be described with reference to Fig. 2. Fig. 2 is a diagram illustrating an image of the reflected light generated in the target object S in a case in which the image acquisition device 1 is used.

Each ray L0 of the polarized light diffused by the illumination device 2 for irradiation widely reaches a surface of the target object S. The target object S such as food has a property of generating specularly reflected light L1 and diffusely reflected light L2 in a case in which the light is incident. In particular, in a case in which the ray L0 of the polarized light is incident on the target object S, a degree of polarization of the specularly reflected light L1 generated based on the ray L0 is relatively high, and a degree of polarization of the diffusely reflected light L2 generated based on the ray L0 is relatively low.

In addition, in a case in which a foreign substance FS such as a plastic film that is an object having a property of transmitting the polarized light is present on the surface of the target object S, the ray L0 of the polarized light incident on the foreign substance FS causes a specularly reflected light L3 that is specularly reflected on the surface of the foreign substance FS, a specularly reflected light L4 that is transmitted through the foreign substance FS and then specularly reflected on a rear surface of the foreign substance FS, and a diffusely reflected light L5 that is diffusely reflected by the foreign substance FS. In this case, the intensity of the specularly reflected light L3 and L4 is relatively higher than the intensity of the specularly reflected light L1, and the degree of polarization of the specularly reflected light L3 and L4 is higher than the degree of polarization of the diffusely reflected light L2 and L5 similarly to the specularly reflected light L1. In addition, in a case in which a material of the foreign substance FS has birefringence, the polarization state of the specularly reflected light L4 is changed from the polarization state of the incident ray L0.

The image processing device 8 of the image acquisition device 1 acquires and stores image data including a plurality of different polarization components or a plurality of pieces of image data corresponding to the plurality of different polarization components, for the same target object S from the camera 6, in order to two-dimensionally detect the reflectivity or the polarization state of the reflected light from the target object S by using the above-described property of the reflected light. Specifically, four or more pieces of image data used for computing a Stokes parameter for each pixel using a rotation compensator method are acquired for the same target object S while the rotating waveplate 4 is being rotated at a predetermined angular interval. In this way, the image processing device 8 acquires the image data indicating the different polarization components by detecting the light including the specularly reflected light that is specularly reflected by the target object S while rotating the rotating waveplate 4.

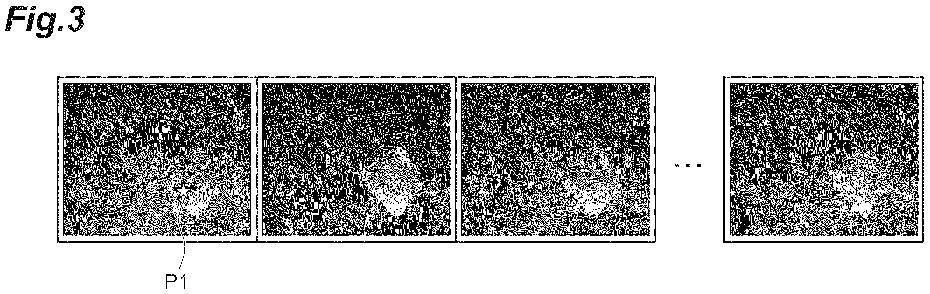

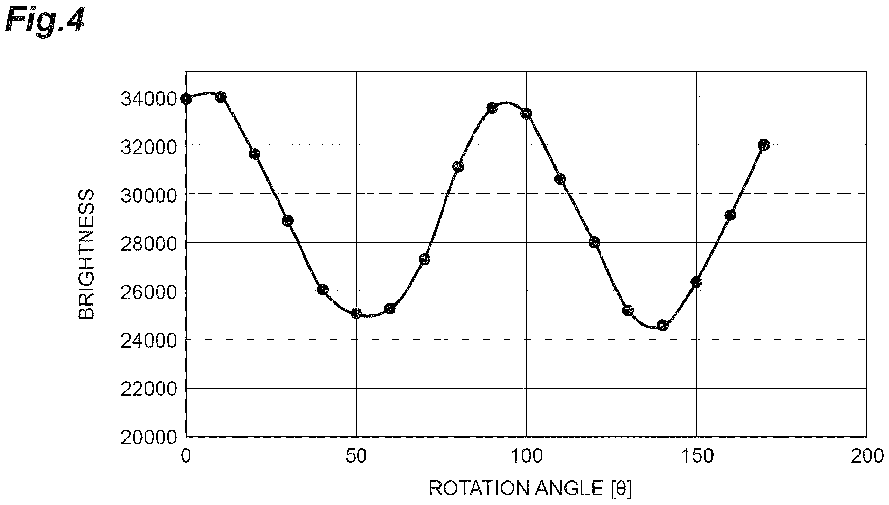

Fig. 3 illustrates an example of a plurality of pieces of image data acquired by the image processing device 8 for the same target object S, and Fig. 4 illustrates a change in brightness in one pixel with respect to a rotation angle of the rotating waveplate 4. In the example illustrated here, 18 pieces of image data are acquired while the rotating waveplate 4 is being rotated at intervals of 10 degrees in a range of 0 degrees to 170 degrees. The graph illustrated in Fig. 4 illustrates the change in brightness at the pixel at a position P1 on the image data illustrated in Fig. 3. As illustrated in the graph, the brightness of the polarization component detected by the pixel is periodically changed by rotating the rotating waveplate 4.

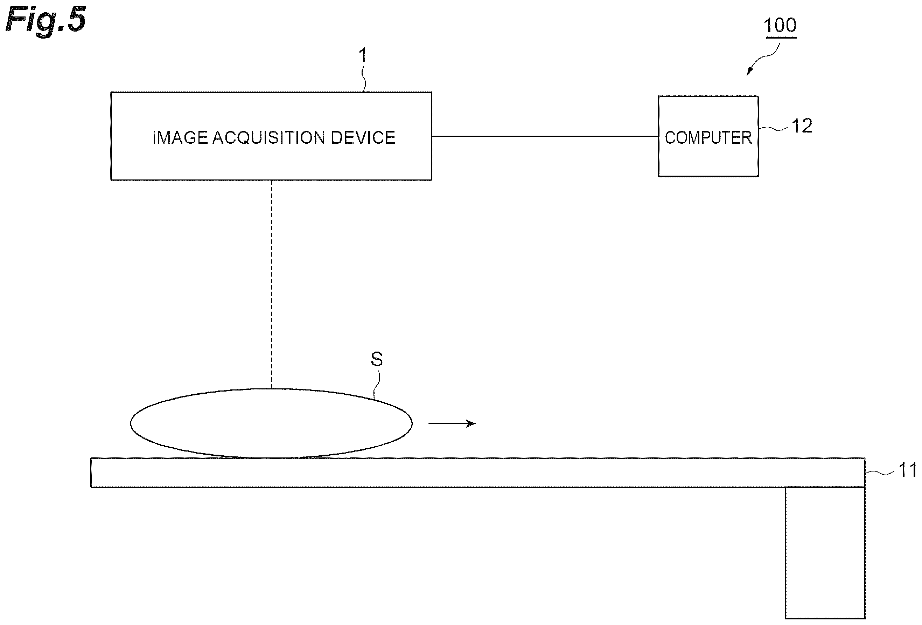

Next, a configuration of an inspection system 100, which is an inspection device according to the embodiment, will be described. Fig. 5 illustrates a schematic configuration of the inspection system 100 according to the embodiment.

The inspection system 100 includes the image acquisition device 1 having the above-described configuration, a transport device **11** such as a belt conveyor that transports the target object S in a predetermined direction, and a computer (inspection processing unit) 12 that performs a computation on the image data output from the image acquisition device 1. The image acquisition device 1 acquires the image data for the target object S transported by the transport device **11** and outputs the acquired image data to the computer 12.

The computer 12 has the same hardware configuration as the image processing device 8. That is, the computer 12 is, physically, a computing device that incorporates a CPU or a GPU as a processor, a RAM or a ROM as a recording medium, a communication module, an input/output module, and the like. The computer 12 may acquire the image data from the image processing device 8 via a cable, or may acquire the image data from the image processing device 8 by wireless communication.

Functionally, the computer 12 executes, for example, inspection processing for the target object S based on the plurality of pieces of image data using the rotation compensator method. That is, the computer 12 first acquires the brightness of one pixel by using the plurality of pieces of image data obtained for the same target object S.

Here, brightness I of the pixel of each image data is theoretically represented by Expression (1).

$$I = I_0(2 + S_1 - 2S_3\sin 2C + S_1\cos 4C + S_2\sin 4C) \ (1)$$

In Expression (1), $I_0$ represents average brightness of all the reflected light incident on the imaging device 7, $S_1$, $S_2$, and $S_3$ represent the Stokes parameters, and C represents the rotation angle of the rotating waveplate 4. The Stokes parameter $S_1$ is a parameter representing a difference in intensity between orthogonal polarization components, the Stokes parameter $S_2$ is a parameter representing a difference in intensity between a polarization component of $+\pi/4$ and a polarization component of $-\pi/4$, and the Stokes parameter $S_3$ is a parameter representing a difference in intensity between a right-handed circularly polarization component and a left-handed circularly polarization component. In addition, a degree of polarization p in the pixel is represented by Expression (2);

$$p = S_1{}^2 + S_2{}^2 + S_3{}^2 \ (2)$$

The computer 12 processes the brightness of one pixel of the plurality of pieces of image data using the relationship of Expression (1), to obtain the Stokes parameters $S_1$, $S_2$, and $S_3$ and the brightness $I_0$ in the pixel by a computation. For example, the computer 12 calculates the Stokes parameters $S_1$, $S_2$, and $S_3$ as Fourier series. Further, the computer 12 repeats the same computation for all pixels of the image data to obtain the Stokes parameters $S_1$, $S_2$, $S_3$ and the brightness $I_0$ for all pixels.

In addition, the computer 12 can obtain an inspection result image showing a distribution of the polarization in the reflected light from the target object S by performing any one of the following computations based on the Stokes parameters $S_1$, $S_2$, $S_3$ and the brightness $I_0$ of each pixel obtained for one target object S.

(Specular reflection distribution) $p \times I_0$
(Linear polarization distribution) $S_1 \times I_0$
(Linear polarization distribution) $S_2 \times I_0$
(Circular polarization distribution) $S_3 \times I_0$

Then, the computer 12 outputs one or more inspection result images acquired for one target object S to an output device such as a display. In addition, the computer 12 may output the inspection result image to an external device through a network, a recording medium, and the like.

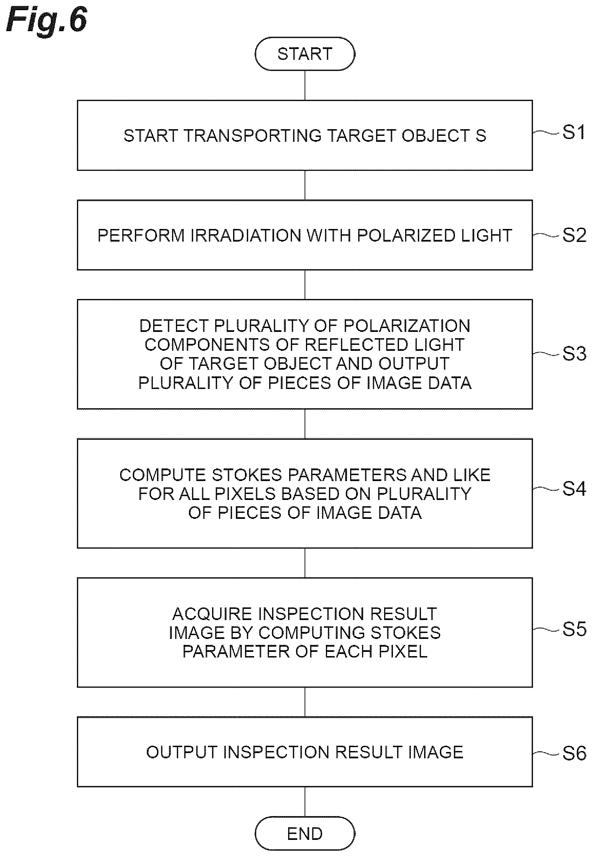

Next, an inspection method for the target object S using the inspection system 100 will be described, and an image acquisition method according to the present embodiment will be described in detail. Fig. 6 is a flowchart illustrating a procedure of the inspection method for the target object S.

First, in a case in which the inspection processing for the target object S is started, the transport of the target object S by the transport device 11 is started (step S1). Then, in a case in which the target object S is transported by the transport device 11 into a range of the irradiation with the polarized light of the illumination device 2, the target object S is irradiated with the polarized light from the illumination device 2 (step S2).

Accordingly, the reflected light generated on the surface of the target object S is incident on the camera 6 via the rotating waveplate 4 and the polarizing plate 5, and the two-dimensional image of the reflected light is detected by the camera 6, so that the image data is output (S3). In this case, the detection of the two-dimensional image of the reflected light is repeated while the rotating waveplate 4 is being rotated, and the plurality of pieces of image data reflecting the detection results of the plurality of polarization components of the reflected light are output.

The image data acquired by the camera 6 is acquired and stored by the image processing device 8, and then is output to the computer 12 and processed by the computer 12. That is, the computer 12 computes the Stokes parameters and the like for each pixel based on the brightness of each pixel of the image data (step S4). Next, the computer 12 acquires one or more inspection result images showing the distribution of the polarization in the reflected light from the target object S by computing the Stokes parameters and the like of each pixel (step S5). The inspection result image is at least one of the specular reflection distribution, the linear polarization distribution, or the circular polarization distribution. At last, the computer 12 outputs the one or more inspection result images for the target object S to the output device as the images in which the foreign substance FS present on the target object S is detected (step S6), and terminates the inspection processing for the target object S.

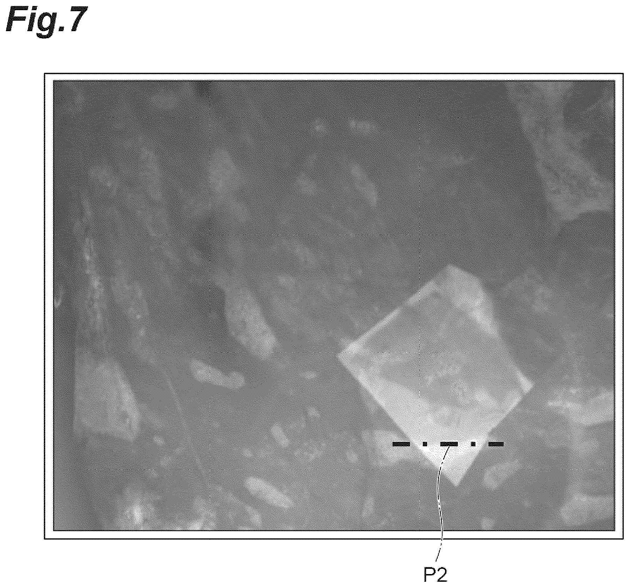

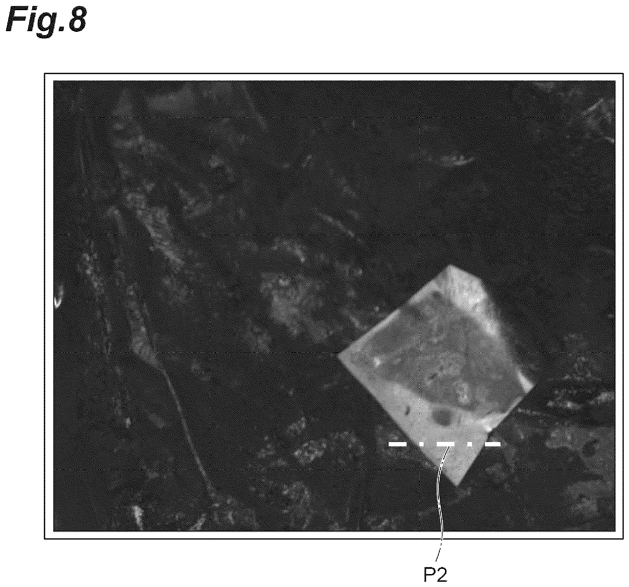

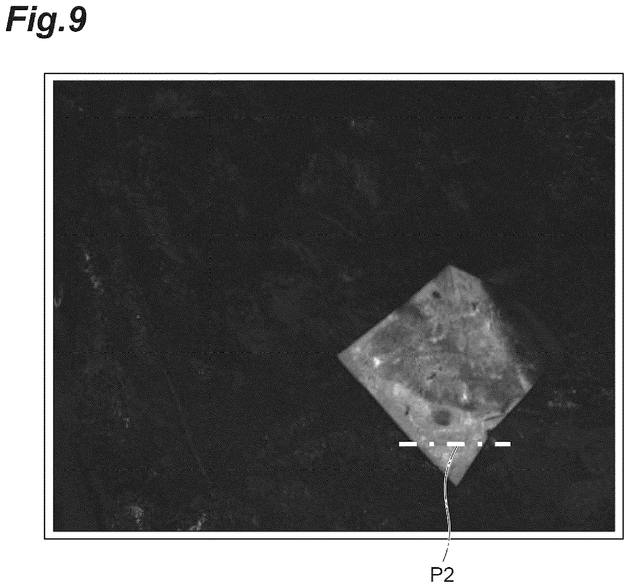

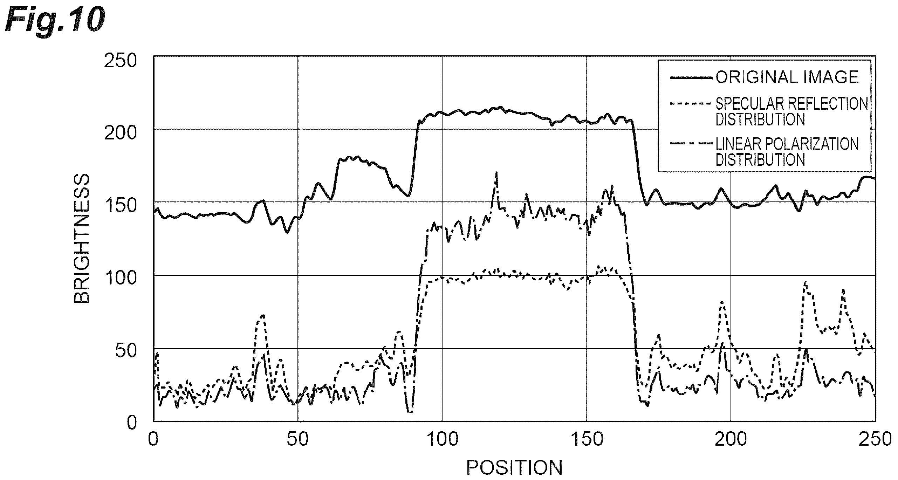

Figs. 7 to 9 illustrate examples of images acquired by the inspection processing of the inspection system 100, Fig. 7 illustrates an original image of the reflected light acquired by the camera 6 in a state in which the rotating waveplate 4 and the polarizing plate 5 are removed, Fig. 8 illustrates an inspection result image that is the specular reflection distribution acquired by the computer 12, and Fig. 9 illustrates an inspection result image that is the linear polarization distribution acquired by the computer 12. Since the illumination light is the circularly polarized light, the linear polarization distribution means an image showing a degree of change in the polarization state. In addition, Fig. 10 is a graph illustrating a brightness distribution at a position of a line P2 on the three images illustrated in Figs. 7 to 9. In a case in which an evaluation value C obtained by dividing the average brightness of the position at which the foreign substance FS is present by the average brightness of the position at which the foreign substance FS is not present is calculated in the brightness distribution, C = 1.5 in a case of Fig. 7, C = 3.3 in a case of Fig. 8, and C = 7.4 in a case of Fig. 9. From this result, it can be seen that the image of the foreign substance FS is embossed in the inspection result image obtained by the inspection system 100, and the foreign substance FS can be effectively detected by the inspection system 100.

According to the present embodiment, it is possible to evaluate the distribution of the polarization states of the light that is specularly reflected by the target object S in the polarized light, based on the image data detected by the image acquisition device 1. As a result, it is possible to detect the foreign substance FS present on the target object S and having the properties of transmitting and reflecting the polarized light with high accuracy.

In the image acquisition device 1, the illumination device 2 is configured to perform irradiation by diffusing the polarized light toward the target object S. As a result, it is possible to detect the foreign substance FS having the properties of transmitting and reflecting the polarized light, in a wide range, in a case in which a three-dimensional target object S such as food is used.

In addition, in the image acquisition device 1, the illumination device 2 is configured to perform the irradiation with light polarized to the circularly polarized light. In this case, even in a case in which the foreign substance FS has the birefringence, it is possible to detect the foreign substance FS with high accuracy.

In addition, in the image acquisition device 1, the imaging device 7 includes the polarizing plate 5 and is configured to detect the reflected light via the polarizing plate 5. In this manner, it is possible to evaluate the distribution of the polarization states of the light that is specularly reflected by the target object S in the polarized light, with a simple configuration.

In addition, in the image acquisition device 1, the imaging device 7 further includes the rotating waveplate 4, and is configured to detect the reflected light via the rotating waveplate 4. In this case, it is possible to accurately evaluate the distribution of the polarization states of the light that is specularly reflected by the target object S in the polarized light by a computation using the Stokes parameters or the like.

Alternatively, with the inspection system 100 according to the present embodiment, it is possible to efficiently detect the objects present on a plurality of target objects S and having the properties of transmitting and reflecting the polarized light while the plurality of target objects S are being transported.

Although various embodiments of the present invention have been described above, the present invention is not limited to the above-described embodiments, and may be modified or applied to other cases without changing the gist described in each claim.

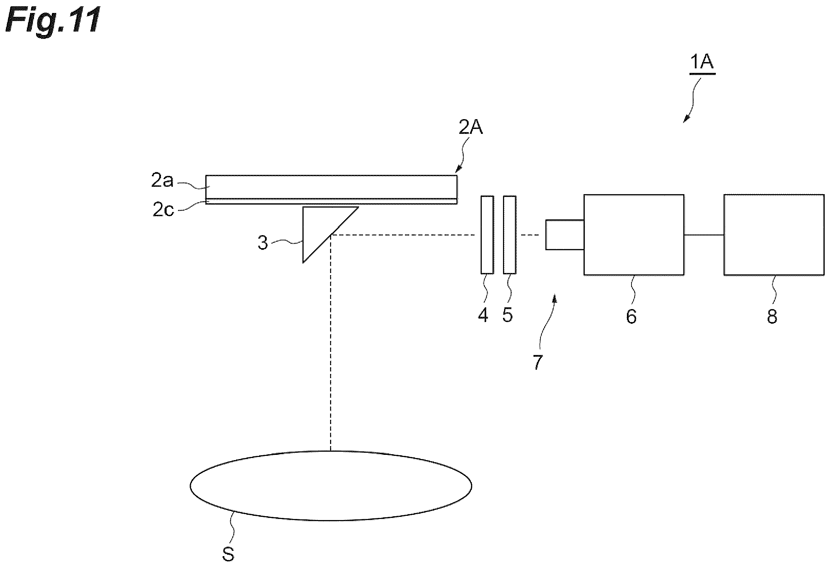

Fig. 11 is a schematic configuration diagram illustrating an image acquisition device 1A according to a first modification example. The image acquisition device 1A has a configuration in which the illumination device 2 in the image acquisition device 1 is replaced with an illumination device 2A. The illumination device 2A is configured by a body portion 2a and a polarizing sheet 2c attached in proximity to a light irradiation surface of the body portion 2a, and performs irradiation by diffusing the polarized light of the linearly polarized light as a predetermined polarization state toward the target object S.

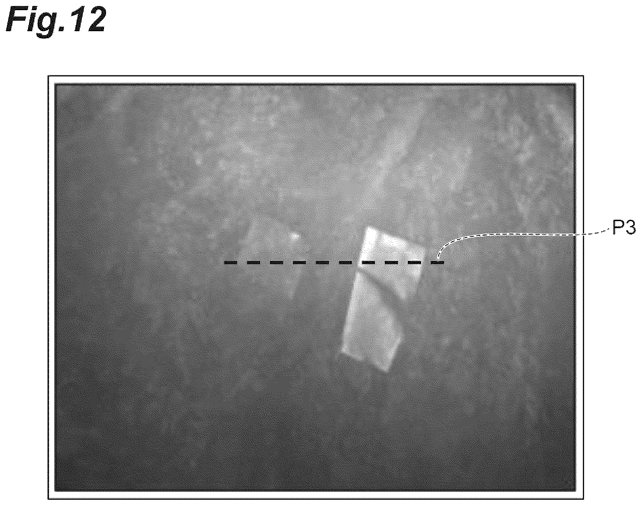

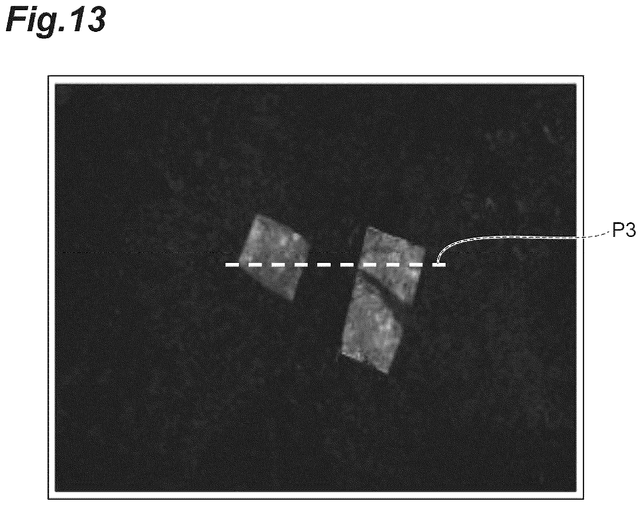

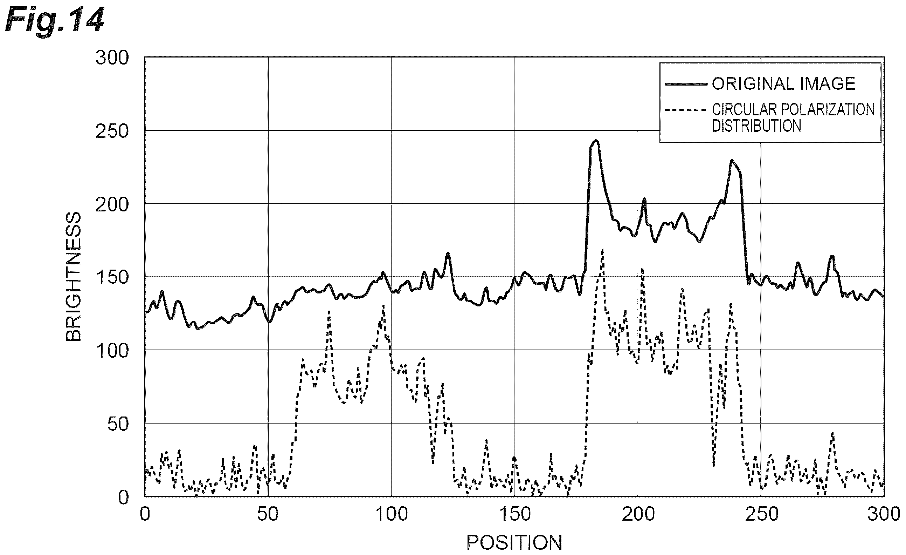

Figs. 12 and 13 illustrate an example of an image acquired by the inspection processing of the inspection system 100 including the image acquisition device 1A, Fig. 12 illustrates an original image of the reflected light acquired by the camera 6 in a state in which the rotating waveplate 4 and the polarizing plate 5 are removed, and Fig. 13 illustrates an inspection result image that is the circular polarization distribution acquired by the computer 12. Since the illumination light is the linearly polarized light, the circular polarization distribution means an image showing a degree of change in the polarization state. In addition, Fig. 14 is a graph illustrating a brightness distribution at a position of a line P3 on the two images illustrated in Figs. 12 and 13. In a case in which the evaluation values C corresponding to the two foreign substances FS are calculated in the brightness distribution, C = 1.1, 1.3 in a case of Fig. 12, and C = 5.9 and 7.1 in a case of Fig. 13. From this result, it can be seen that the image of the foreign substance FS is embossed in the inspection result image obtained by the inspection system 100, and the foreign substance FS can be effectively detected by the inspection system 100.

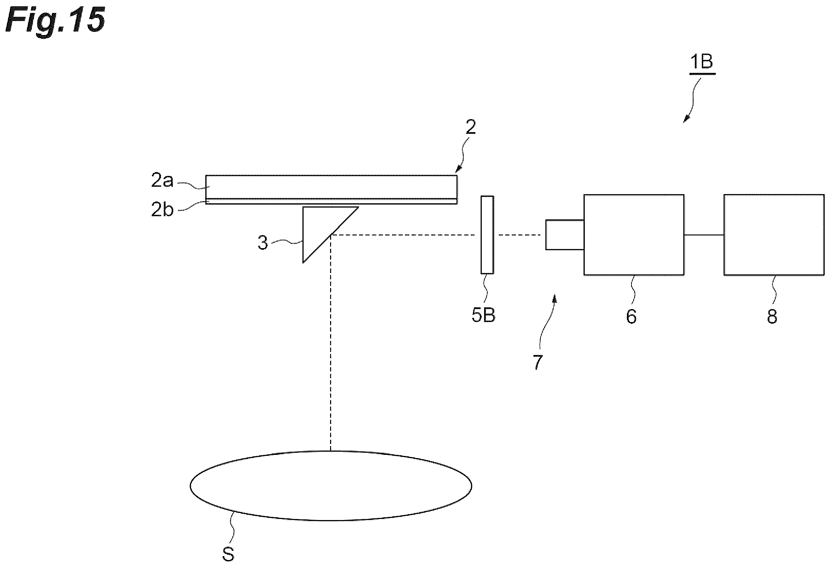

Fig. 15 is a schematic configuration diagram illustrating an image acquisition device 1B according to a second modification example. The image acquisition device 1B has a configuration in which the rotating waveplate 4 and the polarizing plate 5 in the image acquisition device 1 are replaced with a rotating polarizing plate 5B. The rotating polarizing plate 5B is supported to be rotatable with a direction along an incidence direction of the light from the right angle prism mirror 3 as a rotation axis such that a polarization direction of the linearly polarized light to be transmitted is changed. The image processing device 8 of the image acquisition device 1B repeatedly captures two-dimensional images of the target object S by the camera 6 while changing the rotation angle of the rotating polarizing plate 5B for one target object S, and acquires and stores a plurality of pieces of image data output as a result of the capturing. The computer 12 acquires the plurality of pieces of image data acquired by the image processing device 8 as the inspection result images.

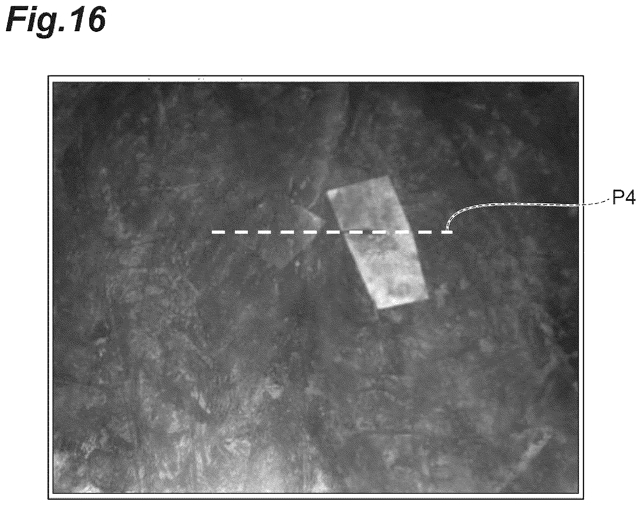

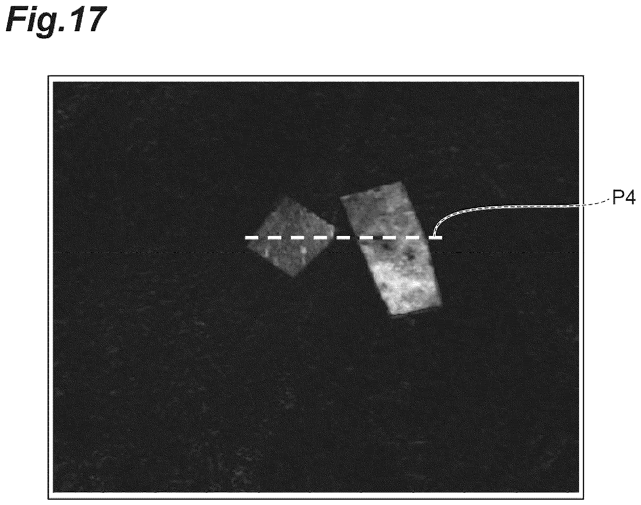

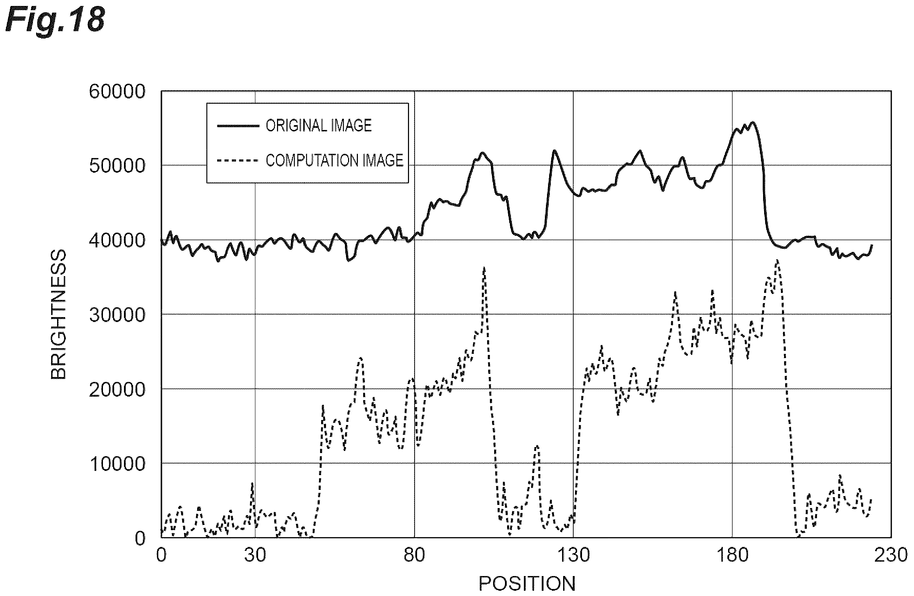

Figs. 16 and 17 illustrate examples of images acquired by the inspection processing of the inspection system 100 including the image acquisition device 1B, Fig. 16 illustrates an original image of the reflected light acquired by the camera 6 in a state in which the rotating polarizing plate 5B is removed, and Fig. 17 illustrates a computation image based on the inspection result image acquired by the computer 12. For example, the computation image can be acquired by subtracting an average value of each of a first inspection result image and a second inspection result image acquired with the rotation angle of the rotating polarizing plate 5B different from each other from each of the first inspection result image and the second inspection result image, and obtaining an absolute value of a difference between the first inspection result image and the second inspection result image in which the average value is subtracted. In addition, Fig. 18 is a graph illustrating a brightness distribution at a position of a line P4 on the two images illustrated in Figs. 16 and 17. In a case in which the evaluation values C corresponding to the two foreign substances FS are calculated in the brightness distribution, C = 1.2 and 1.3 in a case of Fig. 16, and C = 9.2 and 11.4 in a case of Fig. 17. From this result, it can be seen that the image of the foreign substance FS is embossed in the inspection result image obtained by the inspection system 100, and the foreign substance FS can be effectively detected by the inspection system 100.

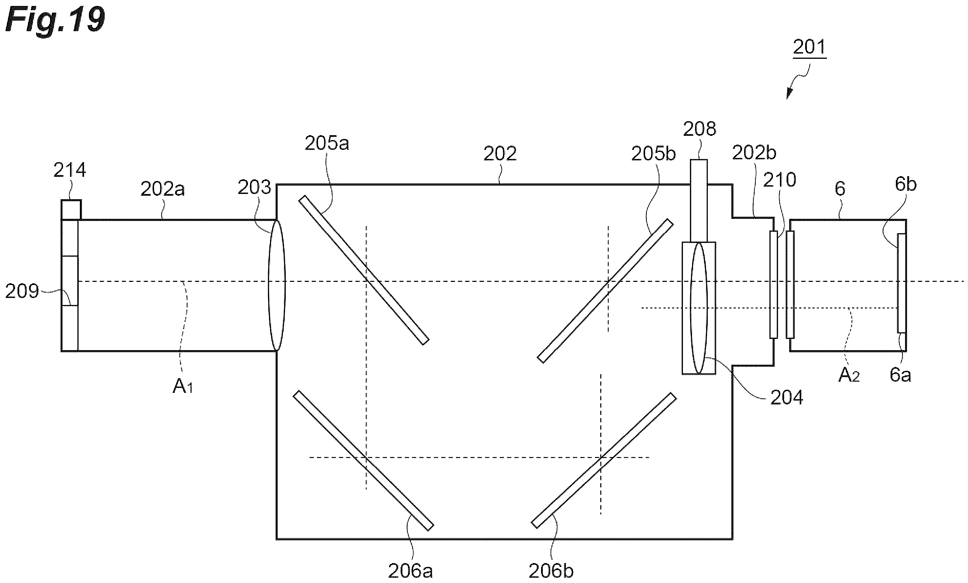

In addition, in the image acquisition device 1B according to the second modification example, the imaging device 201 having the configuration illustrated in Fig. 19 may be used instead of the imaging device 7. The imaging device 201 is configured to split the reflected light of the target object S incident from the right angle prism mirror 3 into a plurality of polarization components and form two-dimensional images of two split polarization components on a light receiving surface of an internal imaging element. Hereinafter, a configuration of the imaging device 201 will be described.

As illustrated in Fig. 19, the imaging device 201 is configured by incorporating a collimator lens 203, an image-forming lens 204, a pre-stage polarizing beam splitter 205a, a post-stage polarizing beam splitter 205b, a pre-stage mirror 206a, a post-stage mirror 206b, and an image-forming lens moving mechanism 208 in a housing 202.

A field-of-view stop 209 having a circular shape is provided at a center portion of an end surface on one end side of a cylindrical portion 202a constituting a part of the housing 202. The field-of-view stop 209 includes a stop adjustment mechanism 214 of which an inner diameter (width) can be set to be variable. The collimator lens 203 is fixed to an inner portion on the other end side of the cylindrical portion 202a such that an optical axis $A_1$ of the collimator lens 203 coincides with a center axis of the cylindrical portion 202a, that is, a center axis of the field-of-view stop 209. With such a structure, the width of the field-of-view stop 209 in a direction perpendicular to the optical axis $A_1$ can be set to be variable. Then, the end surface of the cylindrical portion 202a of the housing 202 is fixed to the right angle prism mirror 3, and the reflected light from the right angle prism mirror 3 is input to the inside of the housing 202 along the optical axis $A_1$ from the field-of-view stop 209. Then, the collimator lens 203 receives the reflected light that passes through the field-of-view stop 209, converts the reflected light into parallel light, and outputs the parallel light to the inside of the housing 202 along the optical axis $A_1$. By

adjusting an aperture of the field-of-view stop 209, it is possible to limit a field-of-view range on an image-forming surface of the reflected light from the target object.

A cylindrical portion 202b that is coaxially located with respect to the cylindrical portion 202a is integrally formed on a side of the housing 202 opposite to the cylindrical portion 202a, and a window portion 210 having a circular shape for allowing a light image formed based on the reflected light input from the cylindrical portion 202a side to pass to the outside is provided in a center portion of an end surface of the cylindrical portion 202b.

Then, the camera 6 is attached to the end surface of the cylindrical portion 202b of the housing 202. The camera 6 incorporates an imaging element 6a, and is attached such that a light receiving surface 6b of the imaging element 6a faces the window portion 210, a center of the light receiving surface 6b is located on the optical axis $A_1$ of the collimator lens 203, and the light receiving surface 6b coincides with an image-forming position of the light image output from the window portion 210. With such an attachment structure, it is possible to capture the light image formed by the imaging device 201 by the camera 6 in a state in which the imaging device 201 is disposed coaxially with respect to the right angle prism mirror 3.

Further, the pre-stage polarizing beam splitter 205a and the post-stage polarizing beam splitter 205b, which are light separation elements, are attachably and detachably disposed on the optical axis $A_1$ inside the housing 202 between the cylindrical portions 202a and 202b. The polarizing beam splitters 205a and 205b are configured to be attachably and detachably provided on the optical axis $A_1$ in the housing 202 by being integrated. The polarizing beam splitters 205a and 205b may also be attachably and detachably provided by being integrated with the pre-stage mirror 206a and the post-stage mirror 206b described below.

The pre-stage polarizing beam splitter 205a is disposed at a position adjacent to the collimator lens 203 such that a center thereof is located on the optical axis $A_1$, and a light receiving surface thereof is inclined by 45 degrees with respect to a plane orthogonal to the optical axis $A_1$. The pre-stage polarizing beam splitter 205a spectrally splits and transmits the linearly polarized light (hereinafter, referred to as "first split light") having a predetermined angle θ1 in the parallel light output from the collimator lens 203, and outputs the first split light along the optical axis $A_1$. At the same time, the pre-stage polarizing beam splitter 205a spectrally splits and reflects the linearly polarized light (hereinafter, referred to as "second split light") having a predetermined angle θ2 different from the predetermined angle θ1 in the parallel light, and outputs the second split light in a direction (down direction in Fig. 19) perpendicular to the optical axis $A_1$.

The post-stage polarizing beam splitter 205b is disposed at a position spaced from the pre-stage polarizing beam splitter 205a toward the window portion 210 side such that a center thereof is deviated from the optical axis $A_1$ by a predetermined distance toward the post-stage mirror 206b side, and a light receiving surface thereof is inclined by 45 degrees with respect to the plane orthogonal to the optical axis $A_1$. The post-stage polarizing beam splitter 205b is configured by an optical member having the same transmission characteristics and reflection characteristics as the pre-stage polarizing beam splitter 205a, and further transmits the first split light transmitted through the pre-stage polarizing beam splitter 205a to output the first split light toward the window portion 210 along the optical axis $A_1$. At the same time, the post-stage polarizing beam splitter 205b reflects the second split light, which is reflected by the pre-stage polarizing beam splitter 205a and then passes through the pre-stage mirror 206a and the post-stage mirror 206b, and outputs the second split light toward the window portion 210 in a direction inclined with respect to the optical axis $A_1$.

That is, the polarizing beam splitters 205a and 205b are light separation elements that separate the parallel light from the collimator lens 203 into two polarization components of the first and second split light.

The pre-stage mirror 206a is provided to be spaced from the pre-stage polarizing beam splitter 205a in a reflection direction of the second split light, and an angle of a light receiving surface of the pre-stage mirror 206a is set to be inclined by 45 degrees with respect to the plane orthogonal to the optical axis $A_1$. The pre-stage mirror 206a reflects the second split light output from the pre-stage polarizing beam splitter 205a in a direction parallel to the optical axis $A_1$. The post-stage mirror 206b is provided to be spaced from the pre-stage mirror 206a in the reflection direction of the second split light and to face the light receiving surface of the post-stage polarizing beam splitter 205b, and an angle of a light receiving surface of the post-stage mirror 206b is set to be inclined by 45 +α degrees (α is a preset angle) with respect to the plane orthogonal to the optical axis $A_1$. The post-stage mirror 206b reflects the second split light, which passes through the pre-stage mirror 206a, toward the light receiving surface of the post-stage polarizing beam splitter 205b in a direction intersecting the optical axis $A_1$.

Further, the image-forming lens 204 supported by the image-forming lens moving mechanism 208 such that the position can be adjusted is provided on the optical axis $A_1$ between the post-stage polarizing beam splitter 205b and the window portion 210. The image-forming lens 204 is supported such that an optical axis $A_2$ is parallel to the optical axis $A_1$, and is configured to move in the direction perpendicular to the optical axis $A_1$ by the image-forming lens moving mechanism 208 while maintaining a state in which the optical axis $A_2$ is parallel to the optical axis $A_1$. Specifically, the image-forming lens 204 is set to a first state in which the optical axis $A_2$ coincides with the optical axis $A_1$ and a second state in which the optical axis $A_2$ is deviated from the optical axis $A_1$ by a predetermined distance, by the image-forming lens moving mechanism 208. In this case, the center of the post-stage polarizing beam splitter 205b is located on the optical axis of the image-forming lens 204. As a result, it is possible to reduce the occurrence of vignetting. The image-forming lens moving mechanism 208 may be a slide mechanism that can continuously adjust the position of the image-forming lens 204, or may be a switching mechanism that switches the position in two stages corresponding to the first and second states. In a case in which the image-forming lens 204 is set to the second state by the image-forming lens moving mechanism 208, the image-forming lens 204 receives the first and second split light split from the reflected light through the polarizing beam splitters 205a and 205b, and forms images of the split light as first and second separated light images on the light receiving surface 6b in the camera 6 attached to the outside of the window portion 210. On the other hand, in a case in which the image-forming lens 204 is set to the first state by the image-forming lens moving mechanism 208, and the polarizing beam splitters 205a and 205b are removed, the image-forming lens 204 receives the reflected light only through the collimator lens 203, and forms images of the reflected light as a single light image on the light receiving surface 6b in the camera 6 attached to the outside of the window portion 210.

The imaging device 201 having such a configuration can be used for both an observation mode in which the reflected light is observed as a single light image by the camera 6 (hereinafter, referred to as a "single view mode") and an observation mode in which the reflected light is separated into two light images and observed by the camera 6 (hereinafter, referred to as a "double view mode"). With the image acquisition device 1B that employs the imaging device 201, it is possible to simultaneously obtain the images of the plurality of polarization components of the reflected light, and it is possible to immediately evaluate the distribution of the polarization states of the light that is specularly reflected by the target object S in the polarized light.

In the above-described embodiment, it is preferable that the light irradiation device performs the irradiation with the polarized light by diffusing the polarized light toward the target object. As a result, it is possible to detect the object having the properties of transmitting and reflecting the polarized light, in a wide range, in a case in which the three-dimensional target object is used.

In addition, in the above-described embodiment, it is also preferable that the light irradiation device performs the irradiation with the polarized light that is circularly polarized light. In this case, even in a case in which the object has the birefringence, it is possible to detect the object with high accuracy.

Further, in the above-described embodiment, it is also preferable that the light irradiation device performs the irradiation with the polarized light that is linearly polarized light. In this manner, it is possible to easily evaluate the distribution of the changes in the polarization state based on the detected image, and it is possible to easily detect the object that transmits and reflects the polarized light.

Furthermore, in the above-described embodiment, it is also preferable that the imaging device includes a polarizing plate, and detects the light that is specularly reflected, via the polarizing plate. In this manner, it is possible to evaluate the distribution of the polarization states of the light that is specularly reflected by the target object in the polarized light, with a simple configuration.

In addition, in the above-described embodiment, it is preferable that the imaging device further includes a waveplate, and detects the light that is specularly reflected, via the waveplate. In this case, it is possible to accurately evaluate the distribution of the polarization states of the light that is specularly reflected by the target object in the polarized light, by a computation.

Further, in the above-described embodiment, it is preferable that the imaging device includes a light separation element that separates the light that is specularly reflected, into a plurality of light components, and the plurality of light components are imaged to acquire the image data including a plurality of images. In this case, it is possible to obtain the plurality of images at the same time, and it is possible to immediately evaluate the distribution of the polarization states of the light that is specularly reflected by the target object in the polarized light.

The image acquisition device according to the embodiment is [1] "An image acquisition device including: a light irradiation device that irradiates a target object with polarized light; an imaging device that detects light that is specularly reflected by the target object in the polarized light, and acquires image data including different polarization components of the light; and an image processing unit that detects an object that is present on the target object and through which the polarized light is transmitted, based on a plurality of images".

The image acquisition device according to the embodiment may be [2] "The image acquisition device according to [1], in which the light irradiation device performs the irradiation with the polarized light by diffusing the polarized light toward the target object".

The image acquisition device according to the embodiment may be [3] "The image acquisition device according to [1] or [2], in which the light irradiation device performs the irradiation with the polarized light that is circularly polarized light".

The image acquisition device according to the embodiment may be [4] "The image acquisition device according to [1] or [2], in which the light irradiation device performs the irradiation with the polarized light that is linearly polarized light".

The image acquisition device according to the embodiment may be [5] "The image acquisition device according to any one of [1] to [4], in which the imaging device includes a polarizing plate, and detects the light that is specularly reflected, via the polarizing plate".

The image acquisition device according to the embodiment may be [6] "The image acquisition device according to [5], in which the imaging device further includes a waveplate, and detects the light that is specularly reflected, via the waveplate".

The image acquisition device according to the embodiment may be [7] "The image acquisition device according to any one of [1] to [4], in which the imaging device includes a light separation element that separates the light that is specularly reflected, into a plurality of light components, and the plurality of light components are imaged to acquire the image data including a plurality of images".

The inspection device according to the embodiment is [8] "An inspection device including: the image acquisition device according to any one of [1] to [7]; a transport device that transports the target object in a predetermined direction; and an inspection processing unit that inspects the target object based on data output from the image acquisition device".

The image acquisition method according to the embodiment is [9] "An image acquisition method including: a light irradiation step of irradiating a target object with polarized light; an imaging step of detecting light that is specularly reflected by the target object in the polarized light, and acquiring image data including different polarization components of the light; and an image processing step of detecting an object that is present on the target object and through which the polarized light is transmitted, based on a plurality of images".

**Reference Signs List**

1, 1A, 1B: image acquisition device
2: illumination device (light irradiation device)
4: rotating waveplate
5: polarizing plate
5B: rotating polarizing plate
7, 201: imaging device
205a, 205b: polarizing beam splitter (light separation element)
8: image processing device (image processing unit)
11: transport device
12: computer (inspection processing unit)
100: inspection system (inspection device)
S: target object
FS: foreign substance

**Claims**

1. An image acquisition device comprising:

   a light irradiation device that irradiates a target object with polarized light;
   an imaging device that detects light that is specularly reflected by the target object in the polarized light, and acquires image data including different polarization components of the light; and
   an image processing unit that detects an object that is present on the target object and through which the polarized light is transmitted, based on the image data.

2. The image acquisition device according to Claim 1,
   wherein the light irradiation device performs the irradiation with the polarized light by diffusing the polarized light toward the target object.

3. The image acquisition device according to Claim 1 or 2,
   wherein the light irradiation device performs the irradiation with the polarized light that is circularly polarized light.

4. The image acquisition device according to Claim 1 or 2,
   wherein the light irradiation device performs the irradiation with the polarized light that is linearly polarized light.

5. The image acquisition device according to any one of Claims 1 to 4,
   wherein the imaging device includes a polarizing plate, and detects the light that is specularly reflected, via the polarizing plate.

6. The image acquisition device according to Claim 5,
wherein the imaging device further includes a waveplate, and detects the light that is specularly reflected, via the waveplate.

7. The image acquisition device according to any one of Claims 1 to 4,
wherein the imaging device includes a light separation element that separates the light that is specularly reflected, into a plurality of light components, and the plurality of light components are imaged to acquire the image data including a plurality of images.

8. An inspection device comprising:

the image acquisition device according to any one of Claims 1 to 7;
a transport device that transports the target object in a predetermined direction; and
an inspection processing unit that inspects the target object based on data output from the image acquisition device.

9. An image acquisition method comprising:

a light irradiation step of irradiating a target object with polarized light;
an imaging step of detecting light that is specularly reflected by the target object in the polarized light, and acquiring image data including different polarization components of the light; and
an image processing step of detecting an object that is present on the target object and through which the polarized light is transmitted, based on the image data.

10. The image acquisition method according to Claim 9,
wherein, in the light irradiation step, the irradiation with the polarized light is performed by diffusing the polarized light toward the target object.

11. The image acquisition method according to Claim 9 or 10,
wherein, in the light irradiation step, the irradiation with the polarized light that is circularly polarized light is performed.

12. The image acquisition method according to Claim 9 or 10,
wherein, in the light irradiation step, the irradiation with the polarized light that is linearly polarized light is performed.

13. The image acquisition method according to any one of Claims 9 to 12,
wherein, in the imaging step, the light that is specularly reflected is detected via a polarizing plate.

14. The image acquisition method according to Claim 13,
wherein, in the imaging step, the light that is specularly reflected is detected via a waveplate.

15. The image acquisition method according to any one of Claims 9 to 12,
wherein, in the imaging step, the light that is specularly reflected is separated into a plurality of light components, and the plurality of light components are imaged to acquire the image data including a plurality of images.

**Fig.1**

# Fig.2

*Fig.3*

Fig.4

EP 4 597 083 A1

**Fig.5**

IMAGE ACQUISITION DEVICE

100

COMPUTER

S

EP 4 597 083 A1

# Fig.6

```
                    ┌─────────────┐
                    │    START    │
                    └─────────────┘
                           │
   ┌───────────────────────────────────────────────┐
   │                                               │
   │   START TRANSPORTING TARGET OBJECT S          │ ~S1
   │                                               │
   └───────────────────────────────────────────────┘
                           │
   ┌───────────────────────────────────────────────┐
   │                                               │
   │   PERFORM IRRADIATION WITH POLARIZED LIGHT     │ ~S2
   │                                               │
   └───────────────────────────────────────────────┘
                           │
   ┌───────────────────────────────────────────────┐
   │       DETECT PLURALITY OF POLARIZATION        │
   │       COMPONENTS OF REFLECTED LIGHT           │
   │       OF TARGET OBJECT AND OUTPUT             │ ~S3
   │     PLURALITY OF PIECES OF IMAGE DATA         │
   └───────────────────────────────────────────────┘
                           │
   ┌───────────────────────────────────────────────┐
   │     COMPUTE STOKES PARAMETERS AND LIKE        │
   │     FOR ALL PIXELS BASED ON PLURALITY         │ ~S4
   │          OF PIECES OF IMAGE DATA              │
   └───────────────────────────────────────────────┘
                           │
   ┌───────────────────────────────────────────────┐
   │          ACQUIRE INSPECTION RESULT            │
   │        IMAGE BY COMPUTING STOKES              │ ~S5
   │          PARAMETER OF EACH PIXEL              │
   └───────────────────────────────────────────────┘
                           │
   ┌───────────────────────────────────────────────┐
   │       OUTPUT INSPECTION RESULT IMAGE          │ ~S6
   └───────────────────────────────────────────────┘
                           │
                    ┌─────────────┐
                    │     END     │
                    └─────────────┘
```

Fig.7

P2

*Fig.8*

P2

*Fig.9*

P2

*Fig.10*

EP 4 597 083 A1

Fig.11

Fig.12

P3

*Fig.13*

Fig.14

Fig.15

*Fig.16*

P4

Fig.17

P4

Fig.18

**Fig.19**

EP 4 597 083 A1

## INTERNATIONAL SEARCH REPORT

| | International application No. |
|---|---|
| | **PCT/JP2023/028775** |

**A. CLASSIFICATION OF SUBJECT MATTER**

**_G01N 21/88_**(2006.01)i; **_G01N 21/89_**(2006.01)i
FI:    G01N21/88 H; G01N21/89 H

According to International Patent Classification (IPC) or to both national classification and IPC

**B. FIELDS SEARCHED**

Minimum documentation searched (classification system followed by classification symbols)

G01N21/84-21/958

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2019-120518 A (HITACHI ZOSEN CORP.) 22 July 2019 (2019-07-22) paragraphs [0015]-[0041], [0053], fig. 1-5 | 1-15 |
| X | JP 2017-134032 A (TOKAN KOGYO CO., LTD.) 03 August 2017 (2017-08-03) paragraphs [0011]-[0036], fig. 1-4 | 1-2, 4-5, 9-10, 12-13 |
| A | WO 2019/151394 A1 (NICHIREI FOODS K.K.) 08 August 2019 (2019-08-08) | 1-15 |
| A | JP 2021-525369 A (LG CHEM, LTD.) 24 September 2021 (2021-09-24) | 1-15 |
| A | KR 10-2154618 B1 (KANG, SIN TAK) 10 September 2020 (2020-09-10) | 1-15 |
| A | US 4262806 A (ELBICON ELECTRONICS PVBA) 21 April 1981 (1981-04-21) | 1-15 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 September 2023** | **10 October 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/028775**

| Patent document cited in search report | | | Publication date (day/month/year) | Patent family member(s) | | | Publication date (day/month/year) |
|---|---|---|---|---|---|---|---|
| JP | 2019-120518 | A | 22 July 2019 | (Family: none) | | | |
| JP | 2017-134032 | A | 03 August 2017 | (Family: none) | | | |
| WO | 2019/151394 | A1 | 08 August 2019 | US | 2021/0035276 | A1 | |
| | | | | CN | 111684268 | A | |
| JP | 2021-525369 | A | 24 September 2021 | US | 2021/0255115 | A1 | |
| | | | | WO | 2019/245313 | A1 | |
| | | | | EP | 3786621 | A1 | |
| | | | | KR | 10-2020-0000221 | A | |
| | | | | CN | 112219114 | A | |
| KR | 10-2154618 | B1 | 10 September 2020 | (Family: none) | | | |
| US | 4262806 | A | 21 April 1981 | GB | 2002899 | A | |
| | | | | DE | 2836025 | A1 | |
| | | | | FR | 2400396 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020190514 A